(19) Europäisches Patentamt / European Patent Office / Office européen des brevets

(11) **EP 3 815 617 A1**

(12) **EUROPEAN PATENT APPLICATION**

(43) Date of publication:
**05.05.2021 Bulletin 2021/18**

(51) Int Cl.:
***A61B 8/08*** (2006.01)

(21) Application number: **19206274.3**

(22) Date of filing: **30.10.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **YANG, Hongxu**
**5656 AE Eindhoven (NL)**

• **KOLEN, Alexander Franciscus**
**5656 AE Eindhoven (NL)**
• **SHAN, Caifeng**
**5656 AE Eindhoven (NL)**
• **DE WITH, Peter Hendrik Nelis**
**5656 AE Eindhoven (NL)**

(74) Representative: **Philips Intellectual Property & Standards**
**High Tech Campus 5**
**5656 AE Eindhoven (NL)**

(54) **IMAGE-PROCESSING METHOD AND APPARATUS FOR OBJECT DETECTION OR IDENTIFICATION**

(57) A method and system for detecting the presence or absence of a target or desired object within a three-dimensional (3D) image. The 3D image is processed to extract one or more 3D feature representations, each of which is then dimensionally reduced into one or more two-dimensional (2D) feature representations. An object detection process is then performed on the 2D features map(s) to generate information about at least the presence or absence of an object within the 2D feature representations, and thereby the overall 3D image.

FIG. 1

EP 3 815 617 A1

**Description**

FIELD OF THE INVENTION

This invention relates to a computer-implemented method and apparatus for object detection or identification within one or more images, such as for identifying and tracking an interventional medical tool.

BACKGROUND OF THE INVENTION

The need for detecting or tracking objects in real time using medical imaging is well known.

Ultrasound imaging is a popular imaging system for tool guidance applications. Ultrasound imaging may be used to image tools such as needles, laparoscopes, stents, and radioactive seeds used for brachytherapy. For example, ultrasound imaging may be used for needle guidance in anesthesiology, tissue ablation or for biopsy guidance, since needles are used to take tissue samples, and to deliver medicine or electrical energy to the targeted tissue inside a patient's body. During these procedures, visualization of the needle and its tip is very important in order to minimize risk to the patient and improve health outcomes.

Object detection is widely applied in other diagnostic and therapy applications, such as for fetal skull segmentation, and cardiac catheter detection for intervention. The object identification may for example be for medical objects such as catheters, guidewires, needles, plugs, etc. as part of a 2D, 3D or 4D ultrasound system. The object identification may instead be for identification or movement tracking of biological tissue such as organs.

For the example of tracking an interventional tool, typically, 2D ultrasound guidance is used to visualize the tool while a procedure is being conducted. However, this mode of imaging has a number of drawbacks for object detection and object tracking. In particular, 2D imaging has a limited field of view; after a successful alignment and localization of the tool in the ultrasound image and while moving the tool or assessing the target, any undesired hand motion of the person conducting the procedure may cause misalignment of the tool and the ultrasound transducer such that parts of the tool are excluded from the ultrasound image. This may lead to incorrect placement of the tool.

Some of the drawbacks of 2D imaging have been tackled by 3D and 4D ultrasound based object detection. However, known 3D and 4D ultrasound based object detection methods require greatly increased processing capability, which make them illsuitable for real-time object detection and object tracking.

Known object detection algorithms are processed in the original spatial coordinate system. For example, 3D ultrasound image-based processing systems for real time identification with short latency provide processing of 3D images in 3D space using an encoder-decoder neural network.

This has high computation complexity and can easily suffer from important restrictions due to inherent hardware limitations. Thus, the application of object identification to 2D images and 3D volumes, and even more particularly for 4D ultrasound volumes, is complicated and expensive for real time applications. The main limitation is that the complex feature processing, i.e. extraction, selection, compression and classification, for a complete image is very time consuming.

The current processing pipelines for object detection in ultrasound images, such as machine learning-based or deep learning-based, mainly focus on the accuracy of object detection, which therefore sometimes introduces a complex algorithm design in full 3D space. As a result, the computation is highly costly to achieve an efficient real-time performance. For interventional ultrasound or ultrasound-based object detection, which requires real time performance, the known processing pipeline is typically not feasible.

There is therefore a need for an improved ultrasound-based object detection system and method, for instance for enabling real-time applications.

SUMMARY OF THE INVENTION

The invention is defined by the claims.

According to examples in accordance with an aspect of the invention, there is provided a computer-implemented method of generating information of a target object within a 3D image.

The computer-implemented method comprises: receiving a 3D image, formed of a 3D matrix of voxels, of a volume of interest; processing the 3D image to generate one or more 3D feature representations; converting each 3D feature representation into at least one 2D feature representation, thereby generating one or more 2D feature representations; detecting an object using the one or more 2D feature representations; and generate information about the detected object.

The proposed invention enables the presence and location of objects within a 3D image to be identified with reduced processing power and/or number of calculations. In particular, the proposed invention effectively decomposes the 3D image into 2D image data, and uses a 2D-image based object recognition process in order to identify objects.

As the 3D image is used to generate the 2D image data, an accuracy of detecting objects can be improved, as the 3D image may contain additional information for overcoming noise, artifacts and/or quality issues with identifying objects in directly obtained 2D image data (e.g. of a 2D image).

By converting a 3D image into 2D feature representations, the information contained in the 3D image can be preserved when detecting an object, whilst enabling use of more efficient (i.e. requiring fewer calculations or parameters) 2D object-detection methodologies. Thus, a significant reduction is made in the number of calculations required to identify an object in the 3D image (compared to a full 3D image-processing methodology).

For the avoidance of doubt, a voxel for a 3D image may contain more than one value (e.g. to represent a value for different channels of the 3D image). Such a construction of a 3D image would be apparent to the skilled person.

A target object is any suitable object, which may be present within the 3D image, about which a user may desire information. The information may comprise, for example, whether the object is present/absent within the 3D image and/or a location, size and/or shape of the object within the 3D image.

A feature representation is a spatial projection of the 3D image, i.e. a partially or fully processed 3D image. A suitable example of a feature representation is a feature map, such as those generated when applying a convolutional neural network to a 3D image.

The step of converting the one or more 3D feature representations may comprise processing each 3D feature representation along a first volumetric axis. A 3D image or feature representation is formed over three volumetric axes. The present invention proposes to generate a 2D feature representation by processing a 3D feature representation over one of these volumetric axis (i.e. along a single dimension or direction).

The step of converting each 3D feature representation may comprise: performing a first process of converting each 3D feature representation into a respective 2D feature representation, by processing each feature representation along a first volumetric axis of the one or more 3D feature representations, to thereby generate a first set of 2D feature representations; and performing a second process of converting each 3D feature representation into a respective 2D feature representation, by processing each feature representation along a second, different volumetric axis of the one or more 3D feature representations, to thereby generate a second set of 2D feature representations.

This enables two sets of 2D feature representations to be generated, along different volumetric axes. Effectively, this results in two sets of feature representations associated with a different plane or projection within the 3D image. This can be used to allow the precise position and orientation of the object within the 3D image (i.e. within the region of interest) to be identified.

The step of generating information about the target object using the one or more 2D feature representations comprises generating first information about the target object using the first set of 2D feature representations and generating second information about the target object using the second set of 2D feature representations.

The step of converting each 3D feature representation into at least one 2D feature representation may comprise generating no more than two 2D feature representations for each 3D feature representation. This helps to minimize the processing performed by the object detection process, whilst enabling accurate detection of a position/location and/or orientation of the object within the 3D image.

The step of generating information about the target object using the one or more 2D feature representations may comprise processing the one or more 2D feature representations using a machine-learning or deep-learning algorithm to generate information about the target object.

In some embodiments, the step of converting each 3D feature representation comprises: performing one or more pooling operations on the 3D feature representation to generate a first 2D feature representation; performing one or more convolution operations on the 3D feature representation to generate a second 2D feature representation; and combining the first and second 2D feature representations to generate the at least one 2D feature representation.

The step of generating one or more 3D feature representations may comprise, for generating each 3D feature representation, performing at least one convolution operation and at least one pooling operation on the 3D image to generate a 3D feature representation.

In some embodiments, the step of receiving a 3D image of a region of interest comprises receiving a 3D ultrasound image. In particular, receiving a 3D image of a region of interest may comprise receiving a 3D ultrasound image obtained using a phased array ultrasound probe.

In some embodiments, the information of the detected object comprises information on a location, shape and/or size of the detected object within the region of interest. In other examples, the information of the detected object comprises an indication of the presence/absence (e.g. a binary indicator) of the object within the 3D image.

In some embodiments, the method further comprises a step of displaying a visual representation of the information on the detected object. Optionally, this step comprises displaying a visual representation of the detected object combined with an image of the volume of interest.

In another aspect of the invention, the object of the invention is also realized by computer program comprising code means for implementing any herein described method when said program is run on a processing system.

According to examples in accordance with an aspect of the invention, there is provided an objection detection system. The object detection system is adapted to: receive a 3D image, formed of a 3D matrix of voxels, of a volume of interest; process the 3D image to generate one or more 3D feature representations; convert each 3D feature representation into at least one 2D feature representation, thereby generating one or more 2D feature representations; generate information about the target object using the one or more 2D feature representations.

Any advantages of the computer-implemented method according to the present invention analogously and similarly apply to the system and computer program herein disclosed.

There is also proposed an ultrasound imaging system comprising: a phase array ultrasound probe adapted to capture ultrasound data and format the ultrasound data into a 3D ultrasound image. The ultrasound imaging system further comprises the object detection system previously described and image visualization module adapted to provide a visual representation of the information about the detected object generated by the object detection system.

These and other aspects of the invention will be apparent from and elucidated with reference to the embodiment(s) described hereinafter.

It will be appreciated by those skilled in the art that two or more of the above-mentioned options, implementations, and/or aspects of the invention may be combined in any way deemed useful.

## BRIEF DESCRIPTION OF THE DRAWINGS

For a better understanding of the invention, and to show more clearly how it may be carried into effect, reference will now be made, by way of example only, to the accompanying drawings, in which:

Figure 1 illustrates an object detection pipeline for understanding an underlying concept of the invention;
Figure 2 illustrates a method of generating 2D feature representations from a 3D image for use in an embodiment of the invention;
Figure 3 illustrates a method of generating a 2D feature representation from a 3D feature representation for use in an embodiment of the invention;
Figure 4 illustrates a method of detecting an object within a 3D feature representation according to an embodiment of the invention;
Figure 5 illustrates an ultrasound imaging system comprising an object detection system according to an embodiment;
Figure 6 is a flowchart illustrating a method according to an embodiment.

## DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method and system for detecting the presence or absence of a target or desired object within a three-dimensional (3D) image. The 3D image is processed to extract one or more 3D feature representations, each of which is then dimensionally reduced into one or more two-dimensional (2D) feature representations. An object detection process is then performed on the 2D features map(s) to generate information about at least the presence or absence of an object within the 2D feature representations, and thereby the overall 3D image.

The underlying inventive concept is based on the realization that the amount of processing power to perform an object-analysis process using 2D data is significantly less than directly performing an object-analysis process using 3D data. The invention thereby proposes to reduce the dimension of 3D data into 2D data (whilst enabling the additional information of 3D data to be preserved), before performing an object detection process. This significantly reduces the processing power required to detect a target object within a 3D image.

Embodiments may be employed to detect the presence or absence of target objects within a 3D ultrasound image, such as the presence or absence of an instrument such as a catheter within a 3D ultrasound image of a patient. Further embodiments may be employed to identify a location, size and/or shape of a target object, such as a catheter, within the 3D image.

The present invention effectively proposes a new object-detection pipeline for detecting the presence or absence of a particular or target object within a 3D image, and optionally determining additional information (such as a location, shape or size) of the target object.

The proposed object-detection pipeline effectively implements a multi-dimension method to identify objects within a 3D image. In particular, an obtained 3D image is partially processed in 3D space and partially processed in 2D space. As a result, the total information processing resources required are reduced (compared to conventional 3D image-processing methodologies), therefore improving the efficiency of processing the 3D image.

Put another way, the invention proposes an object-detection pipeline for performing an object detection task using a dimension-mixed method, which can accelerate the detection efficiency and simplify the algorithm designation or hardware construction.

In particular, the proposed method performs encoding of a 3D image into one or more 3D feature representations using 3D encoding methods, before dimensionally reducing the 3D feature representations into 2D feature representations, which are then decoded determine information about a desired/target object. This effectively enables a 3D-encoding/2D-decoding hybrid concept.

The present invention relies upon a concept of dimensionally reducing 3D data (having dimensions width, height and depth) into 2D data (having only two of these three dimensions). This effectively results in the 3D data being projected into a 2D plane. The reduction may be performed along a single axis or dimension of the 3D data, e.g. along the width dimension/axis, along the height dimension/axis or along the depth dimension/axis.

It will be understood that, if a 2D image is a projection of the 3D image along a particular direction/axis, the 2D image thereby provides information on the 3D image within the other two axes of the 3D image. For example, if a 2D image is produced by reducing a 3D image along a depth dimension, then the produced 2D image will represent the 3D image along the width and height axes (i.e. the dimensions of the 2D image will be "width" and "height").

For ultrasound data, the depth axis of a 3D image typically corresponds to an axial axis (i.e. an axis parallel to a direction of travel of the ultrasound wave during ultrasound imaging). The width axis typically corresponds to either the lateral axis (i.e. an axis perpendicular to a direction of travel of the ultrasound wave during ultrasound imaging) or the elevation axis (i.e. an axis perpendicular to a direction of travel of the ultrasound wave during ultrasound imaging and perpendicular to the lateral axis). The height axis corresponds to the other of the lateral or elevation axes.

Thus, in the context of ultrasound 3D data, a reduction of the 3D data along a depth axis provides an axial projection or "axial view" of the 3D data, whereas a reduction of the 3D data along a width or height axis provides a side projection or "side view" of the 3D data. These definitions will be used later in the description.

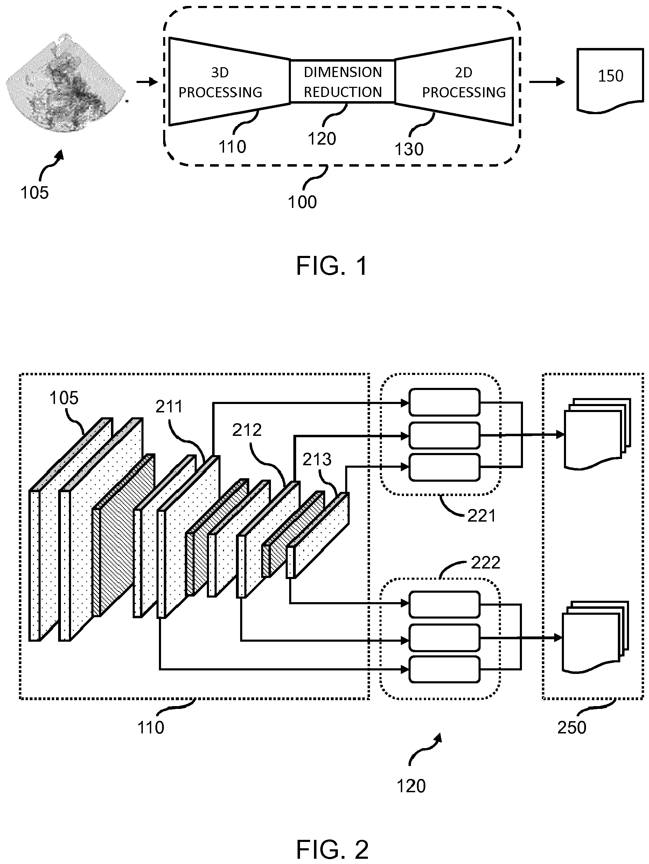

Figure 1 provides a schematic diagram illustrating a proposed object-detection pipeline 100 for performing a method according to an embodiment of the invention.

A 3D image 105 (for example, as illustrated here, a 3D ultrasound image) of a region of interest is the input for the pipeline 100. The 3D image 105 is processed in a step/block 110 to obtain one or more 3D feature representations, which identify features in the 3D image. In particular, the input 3D image is processed in 3D space to explicitly extract features from the whole image.

A feature representation, such as a feature map, is a spatial projection of the 3D image that has been generated by processing the 3D image using one or more non-linear or linear processing steps. In some examples, a feature representation is generated by applying one or more filters to a 3D image. In other examples, a feature representation can be generated by extracting features from the 3D image or by processing a 3D image to form a 3D image with multiple channels (e.g. a hyper-spectrum image). A feature representation may, for example, be a map in which certain elements (e.g. edges, lines, highlights, shadows or voxel groups) of the 3D image are identified or for whom values of corresponding voxels have been modified.

A 3D feature representation is preferably formed of a 3D matrix, each entry in the matrix forming a voxel, each voxel having one or more values (e.g. representing different channels). A 2D feature representation is preferably formed of a 2D matrix, each entry in the matrix forming a pixel, each pixel having one or more value (e.g. representing different channels).

Each one or more 3D feature representation is then converted into one or more 2D feature representations in a step/block 120. Effectively, this is a dimension-reduction block to compress the spatial information (from the 3D feature representation(s)) into a 2D format. This reduces the spatial complexity of the 3D feature representations.

The 2D feature representation(s) are then processed, in a block 130, to identify or detect a (desired/target) object. This may be performed using any suitable 2D image classifier, segmenter and/or detector that is configured to predict the presence/absence and/or location/shape/size of a particular/desired object from a 2D feature representation. In this way, an object within the 3D image can be detected using a multi-dimensional approach.

By way of example only, the 3D image data may comprise a 3D ultrasound image, and block 130 may comprise determining information about a catheter within the 3D image data (and therefore region of interest). The determined information may comprise, for example, information on whether the catheter is present/absent in the 3D image data and/or information on a size, shape and/or location of the catheter within the 3D image data (and therefore within the region of interest).

Information 150 on the detected object may then be output for display. This information may be exploited in a number of ways, and may depend upon a format of the information 150.

In some embodiments, the information comprises a positive or negative prediction of whether a target object is within the 3D image. This information may be presented to a user to enable them to confirm or deny the existence of an object within the 3D image 105. Such embodiments may be useful, for example, if attempting to verify that no objects (e.g. clamps or the like) have been left within a cavity of a patient.

In other embodiments, the information is processed to highlight or identify the predicted location, size and/or shape of the detected object within the 3D image. This may be useful during a surgical procedure to assist a surgeon in guiding the object (e.g. a catheter) within the patient. Specific embodiments of this step will be described later.

Preferably, block 120 comprises a process of reducing a 3D feature representation along a single dimension (e.g. along a height, depth or width of the 3D feature representation) to form a 2D feature representation. The 2D feature representation(s) can then be processed to identify the location of an object within a particular 2D projection direction of the overall 3D image. This information could assist a user to ascertain or understand the approximate location of an object within the 3D image.

In some embodiments, block 120 may comprise generating, for each 3D feature representation, two 2D feature representations including a first feature representation which is a projection or reduction of the 3D feature representation along a first axis and a second feature representation which is a projection or reduction of the 3D feature representation along a second, perpendicular axis.

Where the 3D image is an ultrasound image, the first axis is preferably an axial axis and the second axis is preferably a lateral/elevation axis. This effectively provides two sets of feature representations, a first set associated with an axial view of the 3D image and a second set associated with a side view of the 3D image.

In other words, two sets of 2D feature representations can be produced, a first set comprising the 2D feature representations that are a projection or reduction of a 3D feature representation along the first axis (i.e. provide a view of a 3D feature representation along the first axis), and a second set comprising the 2D feature representations that are a projection or reduction of a 3D feature representation along the second axis. All 2D feature representations in the same set are a projection or reduction of a respective 3D feature representation along/within a same direction with respect to the 3D image.

This effectively generates, for each 3D image, a first set of 2D feature representations associated with a first projection direction of the 3D image and a second set of 2D feature representations associated with a second, different projection direction of the 3D image. The first projection direction is preferably perpendicular to the second projection direction. Each projection direction preferably corresponds to an axis of the 3D image (e.g. width, height or depth).

Each set of 2D feature representations may be processed to identify at least the position of the object with respect to a projection direction of the 3D feature representations (and therefore a projection direction of the 3D image). Once the position of the object within two (perpendicular) projections of the 3D image is known, then the position of the object with the overall 3D image can be ascertained or derived.

By way of example, if the position of an object with respect to a side view of a 3D ultrasound image and with respect to an axial ("top-down") view of the 3D ultrasound image is known, then the overall position of that object within the 3D ultrasound image or region of interest can be derived (as the position of the object with respect to three axes is known). This is the concept employed by the disclosed two set of 2D feature representations approach.

Thus, in some embodiments, no more than two 2D feature representations (each 2D feature representation associated with a different projection direction) need to be generated to enable identification of a location of a target object within a 3D image or region of interest.

The object-detection pipeline of the present invention may be carried out by an object detection system, e.g. comprising one or more processors. The display of any information about a target about may be carried out by one or more user interfaces, e.g. comprising a screen or the like.

Figure 2 to 4 are used to describe a working example of a proposed object-detection pipeline in the context of a deep convolution neural network, which is one possible embodiment of the invention.

However, the object-detection pipeline may be performed using any machine learning or deep learning algorithms to detect the objects, such as artificial neural networks. Other machine-learning algorithms such as logistic regression, support vector machines or Naive Bayesian model are suitable alternatives for forming the object-detection pipeline.

Figure 2 illustrates a schematic diagram for generating one or more 2D feature representations 250 from the input 3D image 105.

The 3D image 105 undergoes a series of convolution operations (indicated using stippling) and pooling steps (indicated using diagonal hatching) to generate one or more feature representations 211-213. As illustrated in Figure 2, a second feature representation 212 may be generated by further processing a first feature representation 211 (e.g. performing additional convolution or pooling steps), and so on.

The 3D feature representations 211-213 are extracted and processed in block 120 to generate the 2D feature representations 250.

In the illustrated example, each 3D feature representation 211-213 is processed to generate two 2D feature representations. In other words, a copy of each 3D feature representation is dimensionally reduced in a first dimension-reducing process 221 to form a first 2D feature representation and is dimensionally reduced in a second dimension-reducing process 222 to form a second 2D feature representation.

Each dimension-reducing process here comprises reducing the dimension of the 3D feature representation with respect to a single direction (e.g. along a single dimension of the 3D feature representation). The first dimension-reducing process 221 may comprise reducing the dimension of the 3D feature representation along an axial direction of the 3D feature representation (e.g. along a depth-direction of the 3D feature representation). The second dimension-reducing process 222 may comprise reducing the dimension of the 3D feature representation from a side of the 3D feature representation (e.g. along a width or height direction of the 3D feature representation).

In particular, for a 3D ultrasound image, a first set of 2D feature representations may be generated providing feature representations from an "axial view" of the 3D ultrasound image (e.g. along an axial axis), and a second set of 2D feature representations may be generated providing feature representations from a "side view" of the 3D ultrasound image (e.g. along a lateral or elevation axis of the 3D image).

The dimensionally reduced 2D feature representations are then further processed to detect information about a target object within the 2D feature representations, such as the presence or absence, and/or a location, shape and/or size of, a target object within the 2D feature representations (and therefore of the overall 3D feature representation and/or image).

In some examples, this process may comprise generating one or more 2D images indicating a location, size and shape of the target object with respect to a certain view of the 3D image (e.g. with respect to a "top-down" view or a "side-view"). In other examples, this process may comprise processing the 2D feature representations to determine whether or not an object is present within the 2D feature representations (and therefore within the overall 3D image).

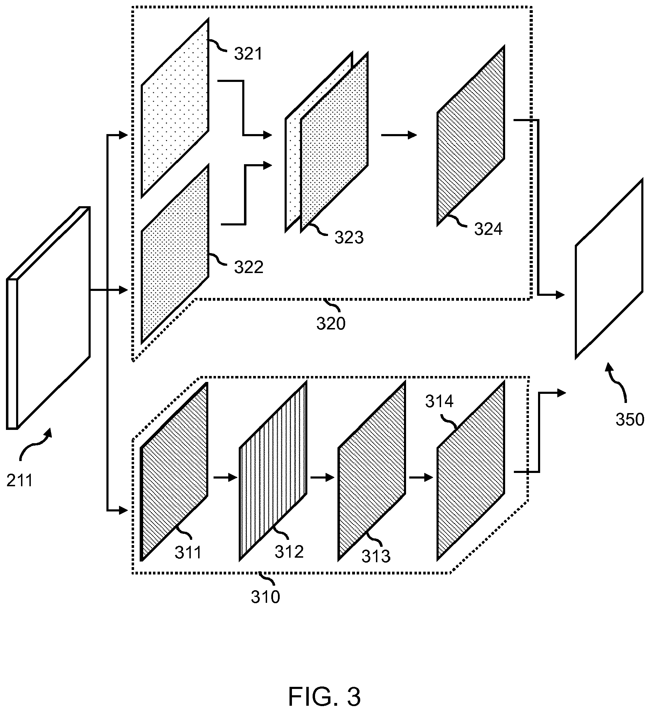

Figure 3 illustrates an example of a suitable dimension-reducing process, for being performed upon a single 3D feature representation 211, for use in embodiments of the invention.

The illustrated dimension-reducing process is effectively a spatiochannel-based attentional process, which is able to extract the most relevant information along a specific dimension and channels, while reducing the size of feature representationpings. The concept of this processing is to summarize the relevant discriminating information along a particular dimension or volumetric axis, which could represent all possible information in that direction. As a result, the illustrated example, the dimension-reducing process is based along one of three principal axes of a 3D feature representation (which dimensions are conventionally width (W), height (H) and depth (D)).

In this working example, the 3D feature representation 211 a 3-dimensional matrix (WxHxD) of voxels, each voxel having a value for each one or more channels (C). Thus, the number of values within the 3D feature representation is equivalent to WxHxDxC. The dimension reduction is performed along one of the volumetric dimensions (width, height or depth), to effectively compress different values along a volumetric dimension into a single value.

The 3D feature representation 211 is processed in two separate paths. A first path 310 comprises applying a series of convolution steps (illustrated with diagonal hatching) to the 3D feature representation. A second path 320 comprises applying at least one pooling step (illustrating using stippling). Different forms of stippling identify different pooling processes. Vertical hatching indicates a reshaping process, which may be required to reduce the dimensionality of a tensor.

In the hereafter described working example, the 3D feature representation is dimensionally reduced along the depth dimension (which is an example of a volumetric axis). This effectively projects the 3D feature representation along the depth dimension or axis. The skilled person would be capable of adapting the illustrated dimension-reducing process for reducing the 3D feature representation along a different dimension (e.g. along the width or height dimension).

The first path 310 comprises performing a series of convolution operations on the 3D feature representation 211 to generate a first depth dimension-reduced feature representation 314. A first convolution operation generates a first tensor 311 of size WxHxDx1, which is reshaped to generate a second tensor 312 of size WxHxD. These two steps effectively compress the channel information of the 3D feature representation. The second tensor 312 is then processed using two further convolution operations: a first to reduce the dimension along the depth axis (generating a third tensor 313 of size WxHx1); and a second to repopulate channel information (generating a fourth tensor 314 of size WxHxC), to thereby obtain a first depth dimension-reduced feature representation.

The second path 320 comprises performing a number of pooling operations on the 3D feature representation 211 to generate a second depth dimension-reduced feature representation that extracts the first-order statistics along the axis of interest (here: the depth axis). This is performed by performing a max(imum) pooling process along the depth axis D to generate a first tensor 321 of size WxHxC and performing an average pooling process along the depth axis D to generate a second tensor 322 of size WxHxC. This maximum pooling process and the average pooling process are performed on their own respective copy of the 3D feature representation 211. This effectively maximizes and averages all possible discriminative information in the depth axis. The first 321 and second 322 tensors are concatenated (to form a third tensor 323 of size WxHx2C) and subsequently processed by a convolution operation to produce a fourth tensor 324 of size WxHxC, thereby generating a second depth-dimension reduced feature representation.

The first and second depth-dimension reduced feature representation 313, 324 from each path is combined, e.g. via an accumulation process, to produce an output 2D feature representation 350.

This illustrated approach is a kind of attention block associated with spatial and channel information, but consisting of different ways to summarize them.

The average pooling step acts to summarize all information along one dimension, while the maximum pooling operation focuses on the maximized signal responses and ignores some minor information. Combining these two features, as performed in the second path 320, enables both forms of information to be taken into account and at least partially preserved when generated the 2D feature representation 250.

The convolution-based path 310 helps to summarize the channel-based information, which acts as a compensation for the above non-parametric approaches.

As a consequence, the information of the 3D feature representation 311 can be summarized appropriately whilst reducing the spatial dimension (here: along the depth axis). In other words, the 3D feature representation can be dimensionally reduced whilst preserving important statistical information contained in the 3D data (which might not be present in a 2D slice of the 3D data).

As schematically illustrated in Figure 2, a dimension-reduction block may be designed for each feature representation, since the number of feature channels is different for each feature representation.

The illustrated method of generating a 2D feature representation from a 3D feature representation is only one example, and the skilled person would be readily capable of adapting an alternative dimension reducing method (e.g. omitting one of the pooling steps or omitting the channel compression steps).

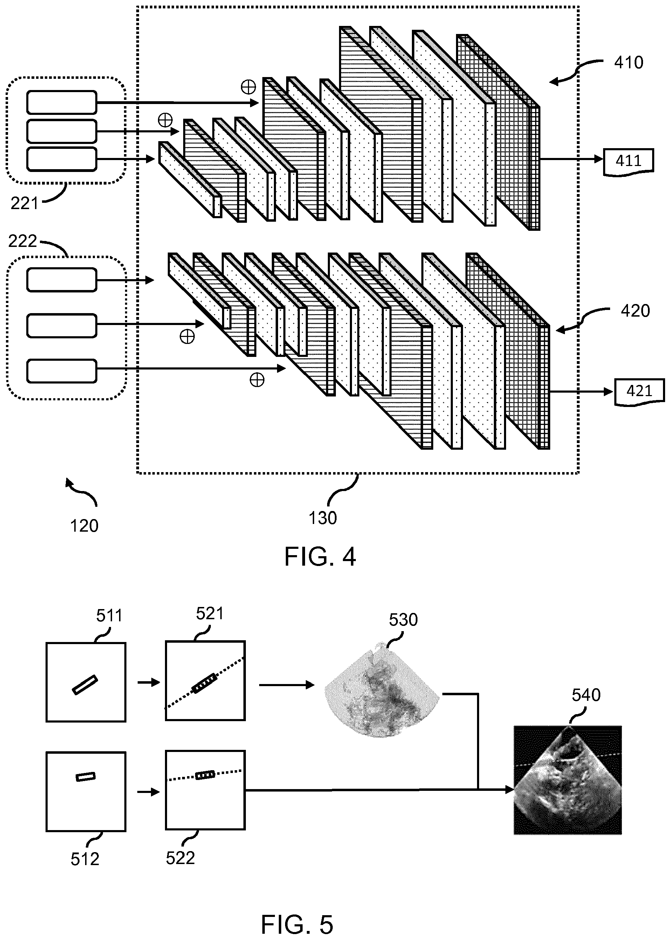

Figure 4 illustrates a 2D decoder 130 according to an embodiment of the invention.

The 2D decoder is designed to generate information about the target object with respect to the 3D image. This may comprise, for example, detecting whether or not an object is present within the 3D image. The 2D decoder may be adapted to generate information about a location, shape and/or size of the target object within the 3D image. In other words, the 2D decoder performs an object detection or recognition process using the 2D feature representations.

In particular examples, the 2D decoder processes the 2D feature representations generated by the 2D conversion block/step 120 to detect the presence or absence of an object within the 2D feature representations (and therefore the overall 3D image). The output of the illustrated 2D decoder is preferably, as illustrated, a prediction of the location, size and shape of the object within a first 2D plane projection of the 3D image and a second 2D plane projection of the 3D image (perpendicular to the first 2D plane).

Thus, the overall object-detection pipeline may output two 2D images 411, 421, each indicating the predicted position of the object within the 3D image with respect to a dimensional projection of the input 3D image. A "dimensional projection" of the 3D image is a projection of the 3D image along one of the dimensions (e.g. height, width or depth) of the 3D image.

In particular, for a 3D ultrasound image, the object-detection pipeline may provide an "axial view" 2D image, being a projection of the 3D ultrasound image along an axial axis (thereby identifying the position and shape of the detected object with respect to the lateral and elevation axes) and a "side view" 2D image, being a projection of the 3D ultrasound image along an lateral/elevation axis (identifying the position and shape of the detected object with respect to the axial and the other of the lateral/elevation axes).

In the illustrated example, two paths are taken by the 2D decoder. A first path 410 generates information about the target object with respect to a first set of 2D feature representations associated with a first projection direction (e.g. an axial view of the 3D image). A second path 420 generates information about the target object with respect to a second set of 2D feature representations associated with a second, different projection direction of the 3D image (e.g. a side view of the 3D image). As previously explained, a first set of 2D feature representations is generated using a first dimension-reducing process 221 and a second set of 2D feature representations is generated using a second dimension-reducing process 222.

The 2D decoder 130 comprises layers that each perform a 2D convolution process followed by a ReLU and Instance Normalization process (each layer being illustrated with stippling). De-convolution layers are applied for upsampling of the 2D feature representations (illustrated with horizontal hatching). A final layer (illustrated with vertical and horizontal hatching) performs a sigmoid operation to predict whether or not a pixel of the 2D feature representation is a pixel of the object to be detected. Each path of the 2D decoder thereby provides an output (2D) image identifying the location and shape of the object with respect to a viewing plane or projection of the 3D image.

Thus, if a first path 410 is associated with an axial view of the 3D image, the output 411 of the first path may illustrate the location of the object with respect to an axial view projection of the region of interest (of the 3D image). Similarly, if a second path 420 is associated with a side view of the region of interest, the output 412 of the second first path may illustrate the location of the object with respect to a side view projection of the 3D image.

The 2D image outputs 411, 412 of the 2D decoder may be used to derive the location of the object within the overall 3D image (and therefore region of interest). This is because the two 2D image outputs provide information on the location of the object with respect to the three axis of the 3D image, thereby enabling the location of the object within the 3D image to be derived.

Some embodiments may comprise providing a visual representation of the 3D image and highlighting the location of the detected object within the 3D image. Of course, if no object is detected within the 3D image, then no identification of the location of the object is provided. This process may be performed by an image visualization module, e.g. which may comprise a screen or the like.

By way of example only, a 3D image can be reconstructed from the 2D image outputs 411, 412 of the 2D decoder. As previously explained, each 2D image output can represent a different view or projection of the original 3D image (e.g. an axial view and a side view), with the position and shape of the detected object being identified.

One particular example of a method for reconstructing the 3D image could be performed by replicating the 2D images along the directions in which they were reduced. For example, if the 3D image was reduced along an axial/depth axis to generate the first output 411 (an "axial view" 2D projection) and along a lateral/width axis to generate the second output (a "side view" 2D projection), then this knowledge may be exploited to reconstruct the 3D image with the location and shape of the identified object being highlights.

As a particular example, a 3D image that enables the shape, size and location of the target object to be identified can be reconstructed by as follows:

$$I_{3D} = Rep(I_{2D-axial}, \theta_{axial}) + Rep(I_{2D-side}, \theta_{side}) \qquad (1)$$

where $Rep(., \theta)$ is the replication operation along the specific direction $\theta$. Based on the reconstructed 3D image $I_{3D}$, a simple threshold and RANSAC model-fitting could be applied on the sparse volume to find the object.

The reconstructed 3D image can then be displayed or output to a user. In some embodiments, the reconstructed 3D image may be superimposed over the original 3D image (provided as input) in order to enable identification of the relative position of the object within the original 3D image.

In another embodiment, the 2D image outputs 411, 412 may be processed to identify a 2D plane of the 3D image containing the object. The 3D image can then be processed to extract image information from that 2D plane, and display it for a user. In this way, the user can be automatically presented with a 2D slice of the 3D image that contains the detected object.

It has been recognized that, in clinical settings, clinicians prefer to view a 2D plane/slice containing an object, rather than a representation of the 3D image containing the object (as a 2D image corresponds to a natural or conventional viewing methodology for a clinician).

For ultrasound data, identifying the plane may be performed by extracting the plane of the 3D image based on the generated axial view 2D image. This exploits the natural property of ultrasound imaging that ultrasound waves always propagate along the direction of the axial direction of the ultrasound probe. The position of the object within the extracted plane of the 3D image can be identified using the generated side view 2D image.

An example of this process is illustrated in Figure 5. The 2D image output by the 2D encoder here comprise a first 2D image 511, providing a 2D axial view/projection of the 3D image, and a second 2D image 512, providing a 2D side view/projection of the 3D image. As previously explained, the 2D side view/projection provides information on the position and shape of an object with respect to an axial axis and a lateral/elevation axis.

Each 2D image is processed to identify a line that passes through the identified object. This may comprise identify a line that passes along a length of the identified object (i.e. along a longest dimension of the identified object).

The processed first 2D image 521 is used to extract a slice 530 of the 3D image. The processed second 2D image 522 is used to identify the location and shape of the detected object within the extracted slice 530 of the 3D image.

The process of identifying a slice 530 of the 3D image can be performed using a single 2D image generated by the object detection pipeline. Thus, in some embodiments, the object detection pipeline is adapted to generate only a single 2D image from the 3D image data (the 2D image identifying a predicted location of the target object with respect to an axial projection of the 3D image). Thus, only a single set of one or more 2D feature representations needs to be generated, as only a single 2D image will be generated.

In yet other embodiments, the output of the 2D decoder is a simple binary indication of whether the object is present within the 3D image, rather than a 2D image output. Thus, rather than constructing 2D images (as illustrated in Figure 4), the 2D feature representation(s) may be processed using a suitable machine-learning algorithm to generate a predictor of whether or not the 2D feature representation(s), and therefore the overall 3D image, contains a desired object.

The skilled person would readily understand other processes that could be performed on one or more 2D feature representations in order to detect information about a (desired) object within the overall 3D image.

As previously explained, Figures 2 to 4 illustrate an implementation of the underlying inventive concept that uses a hybrid deep convolutional neural network in order to predict whether an object is present (and optionally a location of the object) within a 3D image of a region of interest.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output. Examples of these layers have been illustrated in Figures 2 to 4.

Methods of training a machine-learning algorithm are well known. Typically, such methods comprise obtaining a training dataset, comprising training input data entries and corresponding training output data entries (which is often referred to as "ground truth"). An initialized machine-learning algorithm is applied to each input data entry to generate predicted output data entries. An error or "loss function" between the predicted output data entries and corresponding training output data entries is used to modify the machine-learning algorithm. This process can repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error or loss function converges. Known methods of modifying a neural network include gradient descent, backpropagation algorithms and so on.

In the illustrated and previously described examples, the desired input for the proposed neural network is a 3D (volumetric) image, and the desired output comprises a first predicted 2D image that indicates a predicted location, size and shape of the target object with respect to a first view/projection of the 3D image along a first volumetric axis and a second predicted 2D image that indicates a predicted location, size and shape of the target object with respect to a second view/projection of the 3D image along a second volumetric axis. The first and second volumetric axes are preferably perpendicular.

Thus, the corresponding training input data entries comprise example 3D images and the corresponding training output data entries comprise, for each example 3D image, a first ground truth 2D image that indicates a location, size and shape of the target object with respect to a first view/projection of the 3D image along the first volumetric axis, and a second ground truth 2D image that indicates location, size and shape of the target object with respect to a second view/projection of the 3D image along the second volumetric axis.

Where the desired input comprises a 3D ultrasound image, the first volumetric axis is preferably an axial axis and the second volumetric axis is preferably a lateral/elevation axis (so that the second predicted 2D image provides a predicted side view of the 3D image).

To constrain the neural network to improve identification of the position of the object in the predicted 2D image, a multi-level loss function can be used. The aim of training the neural network is to minimize this multi-level loss function. A multi-level loss function may be any suitable loss function that takes account of the prediction of the position of the object in the 2D projected image(s) as well as the high-level description of the object in the 2D projected image(s).

A suitable example of a multi-level loss function is:

$$Loss\left(\hat{Y}_1, \hat{Y}_2, Y_1, Y_2\right) = \alpha Loss_{pixel-level}\left(\hat{Y}_1, \hat{Y}_2, Y_1, Y_2\right) + \beta Loss_{image-level}\left(\hat{Y}_1, \hat{Y}_2, Y_1, Y_2\right) + \qquad (2)$$

$$\gamma \|W\|_2$$

where the loss parameter $Loss_{pixel-level}$ focuses on the prediction of the pixel-wise positon of the object in the predicted 2D image ("pixel-level loss") and $Loss_{image-level}$ focuses upon the high-level description of the object in the predicted 2D image ("image-level loss"). Parameter $\|W\|_2$ denotes the $L$ - 2 regularization for trainable parameters of the neural network.

The term $\hat{Y}_1$ refers to the first ground truth 2D image that that indicates a location, size and shape of the target object with respect to an first view/projection of the 3D image along a first volumetric axis (e.g. along an axial axis) and $\hat{Y}_2$ refers to the second ground truth 2D image that indicates location, size and shape of the target object with respect to a second view/projection of the 3D image along a second volumetric axis. $Y_1$ refers to the predicted 2D image that indicates a predicted location, size and shape of the target object with respect to a first view/projection of the 3D image along a first volumetric axis and $Y_2$ refers to the predicted 2D image that indicates a predicted location, size and shape of the target object with respect to a second view/projection of the 3D image along a second volumetric axis.

More specifically, $Loss_{pixel-level}(\hat{Y}_1, \hat{Y}_2, Y_1, Y_2)$ may be defined as a weighted binary cross entropy specified by

$$Loss_{pixel-level}\left(\hat{Y}_1, \hat{Y}_2, Y_1, Y_2\right)$$

$$= \sum_{j=1}^{N} w_j^{ia} y_j^{ia} \log\left(\hat{y}_j^{ia}\right) \quad (3)$$

$$+ \sum_{j=1}^{N} w_j^{na} y_j^{na} \log\left(\hat{y}_j^{na}\right) + \sum_{j=1}^{N} w_j^{is} y_j^{is} \log\left(\hat{y}_j^{is}\right) + \sum_{j=1}^{N} w_j^{ns} y_j^{ns} \log\left(\hat{y}_j^{ns}\right)$$

where N denotes the number of pixels for each predicted 2D image or corresponding ground truth 2D image, i represents the object pixels and n represents the non-object pixels. y represents a pixel from the predicted 2D image, and $\hat{y}$ identifies the (corresponding) pixel from a ground truth 2D image. Variable a represents the first axis projection, while s denotes the second axis projection. The class weight parameter w is a hyper-parameter to control the weight between two different classes, which is employed because of the extremely imbalance of classes in the ground truth images.

Using an image level loss, $Loss_{image-level}(\hat{Y}_1, \hat{Y}_2, Y_1, Y_2)$, as well as the pixel level loss results in the neural network learning high-level information to properly match the predicted 2D images and the ground truth 2D images.

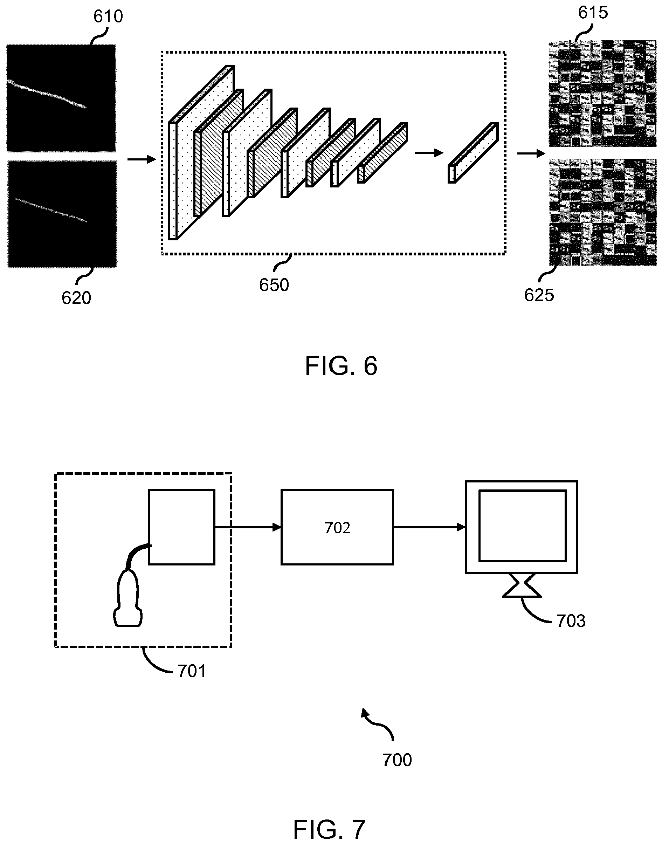

As illustrated in Figure 6, an image level loss may be determined by generating a high-level descriptor 615 of a predicted 2D image 610 and a high-level descriptor 625 the corresponding ground truth 2D image 620, and determining a difference between the high-level descriptors. A high-level descriptor of an image may be generated using a contextual encoder 650, which describes an input image in a latent space. The contextual encoder (CE) receives a 2D image as input, and performs a number of neural network processes on the 2D image to generate a high-level feature representation of the 2D image. In particular, the contextual encoder is a kind of projection function, which projects complex information into a latent high-level space. The feature representations may then be compared to calculate the image level loss.

In the illustrated contextual encoder 650, a convolution process/layer is illustrated using stippling, and a (maximum) pooling process/layer is illustrated with diagonal hatching. For each convolutional layer, ReLU and Instance Normalization layers are also performed to accelerate the convergence.

One method of generating a high-level descriptor (e.g. for calculating an image level loss) is described in H. Yang, C. Shan, T. Tan, A. F. Kolen et al., "Transferring from ex-vivo to in-vivo: Instrument localization in 3d cardiac ultrasound using pyramid-unet with hybrid loss," in International Conference on Medical Image Computing and Computer Assisted Intervention, 2019.

Continuing this example, for each high-level descriptor of each view (i.e. along the first axis or second axis, such as an axial view or side view), the corresponding loss function is defined as the distance between the descriptor of the predicted 2D image and its corresponding ground truth 2D image.

$$Loss_{image-level}\left(\hat{Y}_1, Y_1\right) = \left\|CE\left(\hat{Y}_1\right) - CE(Y_1)\right\|_2 = \|\hat{x} - x\|_2 \quad (4)$$

where parameter CE(.) denotes the high-level descriptor 615, 625 generated by the contextual encoder, $\|.\|_2$ is the norm-2 distance. Equation (3) may be adapted for other predicted views or 2D images of the 3D input image (e.g. the view along the second axis), where appropriate.

$Loss_{image-level}(\hat{Y}_1, \hat{Y}_2, Y_1, Y_2)$ is calculated by summing $Loss_{image-level}(\hat{Y}_1, Y_1)$, calculated using equation (4), and $Loss_{image-level}(\hat{Y}_2, Y_2)$, calculated using an appropriately modified version of equation (4).

The weights $\alpha$, $\beta$, $\gamma$ may be appropriately selected by a skilled person. Examples of suitable weight values may be 1, 0.01 and 0.0001 respectively.

Figure 7 illustrates an ultrasound system 700 according to an embodiment of the invention.

The ultrasound system 700 comprises a phase array ultrasound probe 701 adapted to capture 2D/3D/4D ultrasound data. The data captured by the ultrasound probe 701 is formatted into a 3D ultrasound image.

The ultrasound system further comprises an object detection system 702. The object detection system 702 is adapted to obtain the 3D ultrasound image and process it using any previously described object-detection pipeline for detecting the presence or absence of a (desired) object within the 3D image. The object detection system outputs information 750 on the desired object with respect to the 3D image, e.g. information on whether the desired object is present/absent within the 3D image and/or a location of the desired object within the 3D image.

The ultrasound system 700 further comprises an image visualization module adapted to provide a visual representation of the information 750 output by the object detection system. The visual representation of the information may depend upon the format of the information. For example, if the information comprises a simple indication of the presence/absence of the object within the 3D image, the visual representation may comprise a single light indicating the presence (e.g. when outputting light) or absence (e.g. when not outputting light) of the object. In another example, the information may comprise an indication of the position of the object within the 3D ultrasound image, e.g. by superimposing an indication of the position of the object over a visual representation of the 3D ultrasound image.

The skilled person would be readily capable of developing a method for executing any herein described concept or object detection pipeline.

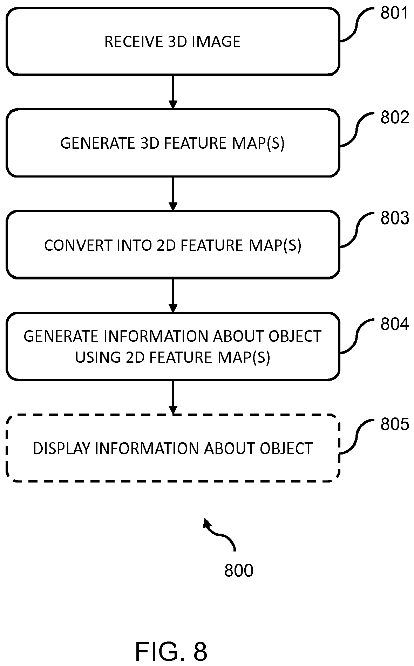

Nonetheless, for the sake of completeness, Figure 8 is a flowchart illustrating a method 800 according to an embodiment of the invention.

The method 800 comprises a step 801 of receiving a 3D image, formed of a 3D matrix of voxels, of a volume of interest; a step 802 of processing the 3D image to generate one or more 3D feature representations; a step 803 of converting each 3D feature representation into at least one 2D feature representation, thereby generating one or more 2D feature representations; a step 804 of detecting an object using the one or more 2D feature representations.

The method may further comprise a step 805 of displaying a visual representation of information on the detected object. This step may be performed by controlling a user interface to provide the visual representation of the information on the detected object.

Step 804 may comprise generating information about the detected object using the one or more 2D feature representations, such as information about the presence/absence of the detected object and/or information on a location and/or shape of the detected object within the 3D image.

Steps 801 to 804 may be carried out by an object detection system, e.g. formed of one or more processors and/or controllers. The 3D image may be received, in step 801, from an ultrasound system and/or a storage facility or memory.

The proposed object detection pipeline could be implemented in ultrasound consoles or on another processing system such as a computer. The 3D image may be a 3D ultrasound image obtained from a phase array adapted to capture Frustum images.

The object detected by the proposed embodiments could comprise any medical item or anatomical tissue/organs, such as catheters, guide wires, cardiac plugs, artificial cardiac valves, valve clips, closure devices, pacemakers, an annuloplasty system, clots, lung nodules, cysts, growths and so on.

It is not essential for the 3D image to be obtained using an ultrasound imaging process, but could rather obtained using any 2D, 3D and 4D imaging modality, such as interventional CT, MRI, or Hyper-spectrum imaging. 2D image data may be stacked in order to generate a 3D image, as is well known in the art.

The term "2D" can be replaced by the term "two-dimensional" where appropriate, and the term "3D" can be replaced by the term "three-dimensional" where appropriate.

Where reference is made to an object, the term "object" may be replaced by "at least one object", e.g. for object detection processes that are able to detect the presence of a plurality of objects within the region of interest.

The skilled person would be readily capable of developing a processing system for carrying out any herein described method. Thus, each step of the flow chart may represent a different action performed by a processing system, and may be performed by a respective module of the processing system.

Embodiments may therefore make use of a processing system. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

It will be understood that disclosed methods are preferably computer-implemented methods. As such, there is also proposed the concept of computer program comprising code means for implementing any described method when said program is run on a processing system, such as a computer. Thus, different portions, lines or blocks of code of a computer program according to an embodiment may be executed by a processing system or computer to perform any herein described method. In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

**Claims**

1. A computer-implemented method (800) for generating information about a target object within a 3D image (105), the method comprising:

   receiving (801) a 3D image (105), formed of a 3D matrix of voxels, of a volume of interest;
   processing (802) the 3D image to generate one or more 3D feature representations (211, 212, 213);
   converting (803) each 3D feature representation into at least one 2D feature representation (250, 350), thereby generating one or more 2D feature representations; and
   generating (804) information (411, 412, 511, 512) about the target object using the one or more 2D feature representations.

2. The computer-implemented method (800) of claim 1, further comprising a step of displaying (805) a visual representation of the information on the target object.

3. A computer-implemented method of claim 1 or 2, wherein the step of converting the one or more 3D feature representations comprises processing each 3D feature representation along a first volumetric axis.

4. The computer-implemented method of claim 1 or 2, wherein the step of converting each 3D feature representation comprises:

   performing a first process of converting each 3D feature representation into a respective 2D feature representation, by processing each feature representation along a first volumetric axis of the one or more 3D feature representations, to thereby generate a first set of 2D feature representations; and
   performing a second process of converting each 3D feature representation into a respective 2D feature representation, by processing each feature representation along a second, different volumetric axis of the one or more 3D feature representations, to thereby generate a second set of 2D feature representations.

5. The computer-implemented method of claim 4, wherein the step of generating information about the target object using the one or more 2D feature representations comprises generating first information (411) about the target object using the first set of 2D feature representations and generating second information (412) about the target object using the second set of 2D feature representations.

**6.** The computer-implemented method of any of claims 1 to 5, wherein the step of converting each 3D feature representation into at least one 2D feature representation comprises generating no more than two 2D feature representations for each 3D feature representation.

**7.** The computer-implemented method of any of claims 1 to 6, wherein the step of generating information about the target object using the one or more 2D feature representations comprises processing the one or more 2D feature representations using a machine-learning or deep-learning algorithm to generate information about the target object.

**8.** The computer-implemented method of any of claims 1 to 7, wherein the step of converting each 3D feature representation comprises:

performing one or more pooling operations on the 3D feature representation to generate a first 2D feature representation;
performing one or more convolution operations on the 3D feature representation to generate a second 2D feature representation; and
combining the first and second 2D feature representations to generate the at least one 2D feature representation.

**9.** The computer-implemented method of any of claims 1 to 8, wherein the step of generating one or more 3D feature representations comprises, for generating each 3D feature representation, performing at least one convolution operation and at least one pooling operation on the 3D image to generate a 3D feature representation.

**10.** A computer-implemented method as claimed in claim 1 to 9, wherein receiving a 3D image of a region of interest comprises receiving a 3D ultrasound image, which is preferably obtained using a phased array ultrasound probe (701).

**11.** A computer-implemented method as claimed in any of claims 1 to 10, wherein the information of the detected object comprises information on a location, shape and/or size of the detected object within the region of interest.

**12.** The computer-implemented method as claimed in any of claims 1 to 11, wherein the step of displaying a visual representation of information on the detected object comprises displaying a visual representation of the detected object combined with an image of the volume of interest.

**13.** A computer program comprising code means for implementing the method of any one of claims 1 to 12 when said program is run on a processing system.

**14.** An objection detection system (703) adapted to:

receive a 3D image, formed of a 3D matrix of voxels, of a volume of interest;
process the 3D image to generate one or more 3D feature representations;
convert each 3D feature representation into at least one 2D feature representation, thereby generating one or more 2D feature representations; and
generate information about the target object using the one or more 2D feature representations.

**15.** An ultrasound imaging system (700) comprising:

a phase array ultrasound probe (701) adapted to capture ultrasound data and format the ultrasound data into a 3D ultrasound image;
the object detection system (702) of claim 14;
and an image visualization module (703) adapted to provide a visual representation of the information about the detected object generated by the object detection system.

FIG. 1

FIG. 2

FIG. 3

FIG. 4

FIG. 5

FIG. 6

FIG. 7

RECEIVE 3D IMAGE — 801

GENERATE 3D FEATURE MAP(S) — 802

CONVERT INTO 2D FEATURE MAP(S) — 803

GENERATE INFORMATION ABOUT OBJECT USING 2D FEATURE MAP(S) — 804

DISPLAY INFORMATION ABOUT OBJECT — 805

800

FIG. 8

Europäisches
Patentamt

European
Patent Office

Office européen
des brevets

# EUROPEAN SEARCH REPORT

Application Number

EP 19 20 6274

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DING MINGYUE ET AL: "Automatic needle segmentation in three-dimensional ultrasound images using two orthogonal two-dimensional image projections", MEDICAL PHYSICS, AIP, MELVILLE, NY, US, vol. 30, no. 2, 2 February 2003 (2003-02-02), pages 222-234, XP012011986, ISSN: 0094-2405, DOI: 10.1118/1.1538231 | 1,3-6, 8-11,13, 14 | INV. A61B8/08 |
| Y | * abstract * <br> * figures 1-7 * <br> * Sections II - VIII * <br> ----- | 7,15 | |
| X | EP 2 363 071 A1 (UNIV EINDHOVEN TECH [NL]) 7 September 2011 (2011-09-07) <br> * abstract * <br> * figures 1-12 * <br> * paragraph [0069] - paragraph [0138] * <br> ----- | 1,2, 12-14 | |
| Y | EP 3 381 512 A1 (KONINKLIJKE PHILIPS NV [NL]) 3 October 2018 (2018-10-03) <br> * abstract * <br> * figures 1-6 * <br> * paragraph [0031] - paragraph [0124] * <br> ----- | 7 | |
| Y | US 2010/121190 A1 (PAGOULATOS NIKOLAOS [US] ET AL) 13 May 2010 (2010-05-13) <br> * abstract * <br> * figures 1-8 * <br> * paragraph [0031] - paragraph [0091] * <br> ----- | 15 | |

TECHNICAL FIELDS
SEARCHED (IPC)

A61B

The present search report has been drawn up for all claims

| Place of search | Date of completion of the search | Examiner |
|---|---|---|
| Munich | 27 April 2020 | Moehrs, Sascha |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 3 815 617 A1**

## ANNEX TO THE EUROPEAN SEARCH REPORT
## ON EUROPEAN PATENT APPLICATION NO.

EP 19 20 6274

27-04-2020

| Patent document cited in search report | | | Publication date | Patent family member(s) | | Publication date |
|---|---|---|---|---|---|---|
| EP 2363071 | A1 | | 07-09-2011 | EP | 2363071 A1 | 07-09-2011 |
| | | | | EP | 2542159 A1 | 09-01-2013 |
| | | | | US | 2012321154 A1 | 20-12-2012 |
| | | | | US | 2016000400 A1 | 07-01-2016 |
| | | | | US | 2017238899 A1 | 24-08-2017 |
| | | | | WO | 2011107404 A1 | 09-09-2011 |
| EP 3381512 | A1 | | 03-10-2018 | CN | 110494188 A | 22-11-2019 |
| | | | | EP | 3381512 A1 | 03-10-2018 |
| | | | | EP | 3600545 A1 | 05-02-2020 |
| | | | | WO | 2018177691 A1 | 04-10-2018 |
| US 2010121190 | A1 | | 13-05-2010 | US | 2010121190 A1 | 13-05-2010 |
| | | | | US | 2014364726 A1 | 11-12-2014 |
| | | | | WO | 2010056560 A1 | 20-05-2010 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **H. YANG ; C. SHAN ; T. TAN ; A. F. KOLEN et al.** Transferring from ex-vivo to in-vivo: Instrument localization in 3d cardiac ultrasound using pyramid-unet with hybrid loss. *International Conference on Medical Image Computing and Computer Assisted Intervention,* 2019